# EUROPEAN PATENT APPLICATION

(11) **EP 4 130 257 A1**
(43) Date of publication of application: **08.02.2023**
(21) Application number: 21764693.4
(22) Date of filing: 04.03.2021
(51) Int. Cl.: C12N 15/00, C12N 9/22, C07K 14/00

(54) **IMPROVED CYTOSINE BASE EDITING SYSTEM**

(30) Priority: 04.03.2020 CN 202010145047
(71) Applicant: Suzhou Qi Biodesign biotechnology Company Limited, Jiangsu 215127 (CN)
(72) Inventor: GAO, Caixia, Beijing 100101 (CN); WANG, Yanpeng, Beijing 100101 (CN); JIN, Shuai, Beijing 100101 (CN); ZONG, Yuan, Beijing 100101 (CN)
(74) Representative: Michalski Hüttermann & Partner Patentanwälte mbB
(86) International application number: PCT/CN2021/079086
(87) International publication number: WO 2021/175288

(57) **Abstract**

The present invention belongs to the field of gene editing. In particular, the present invention relates to an improved cytosine base editing system which has a significantly reduced genome-wide off target effect and a narrow editing window.

## Description

### Technical Field

The present invention belongs to the field of gene editing. In particular, the present invention relates to an improved cytosine base editing system which has a significantly reduced genome-wide off target effect and a narrow editing window.

### Background Art

Gene editing technology is a gene engineering technology used for targeted modification of a genome based on an artificial nuclease, which plays an increasingly powerful role in agricultural and medical research. Currently, clustered regularly interspaced short palindromic repeats/CRISPR associated system is the most widely used genome editing tool, and Cas protein can target any positions in the genome under the guidance action of guide RNA. Base editing systems are novel gene editing technology developed based on the CRISPR system, including cytosine base editing systems and adenine base editing systems respectively fusing a cytosine deaminase and s adenine deaminase with a Cas9 single-stranded nickase. Under the targeting action of guide RNA, a single-stranded DNA region is formed by the Cas9 single-stranded nickase, and therefore the deaminase can efficiently and respectively remove amino groups of C and A nucleotides on single-stranded DNA at a targeting position to become U base and I base which are then repaired into T base and G base in the self-repairing process of cells.

The cytosine base editing system is found to create an unpredicted off target phenomenon in the genome, which may be caused by a random deamination phenomenon generated in a high transcriptional active region in the genome due to overexpression of cytosine deaminase in the genome. In addition, if there are multiple C in the working window of a target site, the existing efficient base editing system can often obtain a product where multiple C are simultaneously changed instead of a product where only a single C is mutated. The specificity in the genome and accuracy at the target site greatly affect the application of the cytosine base editing system.

### Summary of the Invention

The specificity and accuracy of the cytosine base editing system both may be associated with the binding ability of cytosine deaminase to single-stranded DNA. Changing or impairing the binding ability of deaminase to single-stranded DNA while not reducing the deamination ability of the deaminase may obtain a cytosine base editing system that is not only efficient but also simultaneously has specificity and accuracy. Through optimization of Loop1 and Loop7 in thehuman-derived hA3Bctd domain (APOBEC3B C-terminal domain) which binds to single-stranded DNA and by testing the obtained mutants via rice protoplast transformation, the inventors detect the efficiency and accuracy of obtaining the mutants and test the specificity of the obtained mutants, thereby obtaining a series of base editing systems with high-efficiency, high-specificity, and high-accuracy.

### Brief Description of the Drawings

Fig.1 shows the selection of A3Btd mutation sites.
Fig.2 shows on-target efficiency and off-target efficiency of a to-be-tested base editing system.
Fig.3 shows average on target efficiency and average off target efficiency of the to-be-tested base editing system.
Fig.4 shows combination of double mutants and triple mutants.
Fig.5 shows verification of on-target efficiency and off-target efficiency of double mutants and triple mutants through protoplast transformation.
Fig.6 shows average on-target efficiency and average off-target efficiency of to-be-tested double mutants and triple mutants.
Fig.7 shows working efficiencies on different C of different base editing systems at four target sites.
Fig.8 shows average mutation types in editing products of different base editing systems at four target sites.

### Detailed Description of the Invention

### I. Definition

In the present invention, unless indicated otherwise, the scientific and technological terminologies used herein refer to meanings commonly understood by a person skilled in the art. Also, the terminologies and experimental procedures used herein relating to protein and nucleotide chemistry, molecular biology, cell and tissue cultivation, microbiology, immunology, all belong to terminologies and conventional methods generally used in the art. For example, the standard DNA recombination and molecular cloning technology used herein are well known to a person skilled in the art, and are described in details in the following references: Sambrook, J., Fritsch, E.F.and Maniatis, T., Molecular Cloning: A Laboratory Manual; Cold Spring Harbor Laboratory Press: Cold Spring Harbor, 1989. In the meantime, in order to better understand the present invention, definitions and explanations for the relevant terminologies are provided below.

As used herein, the term "and/or" encompasses all combinations of items connected by the term, and each combination should be regarded as individually listed herein. For example, "A and/or B" covers "A", "A and B", and "B". For example, "A, B, and/or C" covers "A", "B", "C", "A and B", "A and C", "B and C", and "A and B and C".

When the term "comprise" is used herein to describe the sequence of a protein or nucleic acid, the protein or nucleic acid may consist of the sequence, or may have additional amino acids or nucleotide at one or both ends of the protein or nucleic acid, but still have the activity described in this invention. In addition, those skilled in the art know that the methionine encoded by the start codon at the N-terminus of the polypeptide will be retained under certain practical conditions (for example, when expressed in a specific expression system), but does not substantially affect the function of the polypeptide. Therefore, when describing the amino acid sequence of specific polypeptide in the specification and claims of the present application, although it may not include the methionine encoded by the start codon at the N-terminus, the sequence containing the methionine is also encompassed, correspondingly, its coding nucleotide sequence may also contain a start codon; vice versa.

As used herein, the term "CRISPR effector protein" generally refers to nuclease existing in a naturally occurring CRISPR system, and modified forms, variants, catalytically active fragments and the like thereof. The term covers any effector protein based on the CRISPR system and capable of achieving gene targeting (such as gene editing and targeted gene regulation) in cells.

Examples of the "CRISPR effector protein" include Cas9 nuclease or a variant thereof. The Cas9 nuclease can be Cas9 nuclease from different species, such as spCas9 from S. pyogenes or SaCas9 derived from S. aureus. The terms "Cas9 nuclease" and the "Cas9" can be used interchangeably in the present invention, and refer to a RNA-guided nuclease comprising a Cas9 protein or a fragment thereof (such as a protein comprising an active DNA cleavage domain of Cas9 and/or a gRNA binding domain of Cas9). Cas9 is a component of a CRISPR/Cas (Clustered regularly interspaced short palindromic repeats/CRISPR associated) genome editing system, and can target and cleave a DNA target sequence to form a DNA double-strand break (DSB) under the guidance of guide RNA.

The examples of the "CRISPR effector protein" can further comprise Cpfl nuclease or a variant thereof, such as a high-specificity variant. The Cpfl nuclease can be Cpfl nuclease from different species, such as Cpfl nuclease from Francisella novicida U112, Acidaminococcus sp. BV3L6 and Lachnospiraceae bacterium ND2006.

"CRISPR effector protein" can also be derived from Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Csn2, Cas4, C2c1, C2c3 or C2c2 nucleases, for example, include these nucleases or functional variants thereof.

"Genome" as used herein encompasses not only chromosomal DNA present in the nucleus, but also organelle DNA present in the subcellular components (e.g., mitochondria, plastids) of the cell.

As used herein, "organism" includes any organism that is suitable for genome editing, eukaryotes are preferred. Examples of organisms include, but are not limited to, mammals such as humans, mice, rats, monkeys, dogs, pigs, sheep, cattle, cats; poultry such as chickens, ducks, geese; plants including monocots and dicots such as rice, corn, wheat, sorghum, barley, soybean, peanut, Arabidopsis and the like.

A "genetically modified organism" or "genetically modified cell" includes the organism or the cell which comprises within its genome an exogenous polynucleotide or a modified gene or expression regulatory sequence. For example, the exogenous polynucleotide is stably integrated within the genome of the organism or the cell such that the polynucleotide is passed on to successive generations. The exogenous polynucleotide may be integrated into the genome alone or as part of a recombinant DNA construct. The modified gene or expression regulatory sequence means that, in the organism genome or the cell genome, said sequence comprises one or more nucleotide substitution, deletion, or addition.

The term "exogenous" with respect to sequence means a sequence that originates from a foreign species, or, if from the same species, is substantially modified from its native form in composition and/or genomic locus by deliberate human intervention.

"Polynucleotide", "nucleic acid sequence", "nucleotide sequence", or "nucleic acid fragment" are used interchangeably to refer to a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation. Suitable conserved amino acid replacements in peptides or proteins are known to those skilled in the art and can generally be carried out without altering the biological activity of the resulting molecule. In general, one skilled in the art recognizes that a single amino acid replacement in a non-essential region of a polypeptide does not substantially alter biological activity (See, for example, Watson et al., Molecular Biology of the Gene, 4th Edition, 1987, The Benjamin/Cummings Pub. co., p.224).

As used herein, an "expression construct" refers to a vector suitable for expression of a nucleotide sequence of interest in an organism, such as a recombinant vector. "Expression" refers to the production of a functional product. For example, the expression of a nucleotide sequence may refer to transcription of the nucleotide sequence (such as transcribe to produce an mRNA or a functional RNA) and/or translation of RNA into a protein precursor or a mature protein.

"Expression construct" of the invention may be a linear nucleic acid fragment, a circular plasmid, a viral vector, or, in some embodiments, an RNA that can be translated (such as an mRNA).

"Expression construct" of the invention may comprise regulatory sequences and nucleotide sequences of interest that are derived from different sources, or regulatory sequences and nucleotide sequences of interest derived from the same source, but arranged in a manner different than that normally found in nature.

"Regulatory sequence" or "regulatory element" are used interchangeably and refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a nucleic acid fragment capable of controlling the transcription of another nucleic acid fragment. In some embodiments of the present invention, the promoter is a promoter capable of controlling the transcription of a gene in a cell, whether or not it is derived from the cell. The promoter may be a constitutive promoter or a tissue-specific promoter or a developmentally-regulated promoter or an inducible promoter.

"Constitutive promoter" refers to a promoter that may cause expression of a gene in most circumstances in most cell types. "Tissue-specific promoter" and "tissue-preferred promoter" are used interchangeably, and refer to a promoter that is expressed predominantly but not necessarily exclusively in one tissue or organ, but that may also be expressed in one specific cell or cell type. "Developmentally regulated promoter" refers to a promoter whose activity is determined by developmental events. "Inducible promoter" selectively expresses a DNA sequence operably linked to it in response to an endogenous or exogenous stimulus (environment, hormones, or chemical signals, and so on).

As used herein, the term "operably linked" means that a regulatory element (for example but not limited to, a promoter sequence, a transcription termination sequence, and so on) is associated to a nucleic acid sequence (such as a coding sequence or an open reading frame), such that the transcription of the nucleotide sequence is controlled and regulated by the transcriptional regulatory element. Techniques for operably linking a regulatory element region to a nucleic acid molecule are known in the art.

"Introduction" of a nucleic acid molecule (e.g., plasmid, linear nucleic acid fragment, RNA, etc.) or protein into an organism means that the nucleic acid or protein is used to transform a cell of the organism such that the nucleic acid or protein functions in the cell. As used in the present invention, "transformation" includes both stable and transient transformations.

"Stable transformation" refers to the introduction of an exogenous nucleotide sequence into the genome, resulting in the stable inheritance of foreign genes. Once stably transformed, the exogenous nucleic acid sequence is stably integrated into the genome of the organism and any of its successive generations.

"Transient transformation" refers to the introduction of a nucleic acid molecule or protein into a cell, performing its function without the stable inheritance of an exogenous gene. In transient transformation, the exogenous nucleic acid sequence is not integrated into the genome.

"Trait" refers to the physiological, morphological, biochemical, or physical characteristics of a cell or an organism.

"Agronomic trait" is a measurable parameter including but not limited to, leaf greenness, yield, growth rate, biomass, fresh weight at maturation, dry weight at maturation, fruit yield, seed yield, total plant nitrogen content, fruit nitrogen content, seed nitrogen content, nitrogen content in a vegetative tissue, total plant free amino acid content, fruit free amino acid content, seed free amino acid content, free amino acid content in a vegetative tissue, total plant protein content, fruit protein content, seed protein content, protein content in a vegetative tissue, drought tolerance, nitrogen uptake, root lodging, harvest index, stalk lodging, plant height, ear height, ear length, disease resistance, cold resistance, salt tolerance, and tiller number and so on.

### II. Improved base editing system

First, the present invention provides a base editing fusion protein, comprising an APOBEC3B deaminase or a APOBEC3B deaminase mutant fused with a CRISPR effector protein.

In the embodiments herein, "base editing fusion protein" and "base editor" can be used interchangeably. The base editing fusion protein comprising the APOBEC3B deaminase or mutant thereof can perform efficient base editing on a target sequence, and meanwhile has a significantly reduced genome-wide random off-target effect compared with other base editors. In some embodiments, the base editing fusion protein comprising the APOBEC3B deaminase or mutant thereof has a shortened editing window in the target sequence, and is capable of realizing more precise base editing.

In some embodiments, the APOBEC3B deaminase mutant is or is derived from a human APOBEC3B deaminase. An exemplary wild-type APOBEC3B deaminase comprises an amino acid sequence as shown in SEQ ID NO: 19.

In some embodiments, the APOBEC3B deaminase mutant is or is derived from a C-terminal domain (hA3Bctd, APOBEC3B C-terminal domain) of human APOBEC3B deaminase. An exemplary hA3Bctd comprises an amino acid sequence as shown in SEQ ID NO:2.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at one or more of position 210, position 211, position 214, position 230, position 240, position 281, position 308, position 311, position 313, position 314 and position 315 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at one or more of position 211, position 214, position 308, position 311, position 313, position 314 and position 315 relative to wild-type hA3B or hA3Bcrd, wherein the amino acid position is determined by reference to SEQ ID NO: 19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 211 and position 311 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 211 and position 313 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 211 and position 314 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 311 and position 313 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 214 and position 314 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 314 and position 315 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 211, position 311 and position 314 relative to wild-type hA3B or hA3Bcrd, wherein the amino acid position is determined by reference to SEQ ID NO: 19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 211, position 214 and position 313 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions at position 214, position 314 and position 315 relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises one or more amino acid substitutions selected from R210A, R210K3, R211K, T214C, T214G, T214S, T214V, L230K, N240A, W281H, F308K, R311K, Y313F, D314R, D314H and Y315M relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises one or more amino acid substitutions selected from R211K, T214V, F308K, R311K, Y313F, D314R, D314H and Y315M relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions R211K and R311K relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions R211K and Y313F relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions R211K and D314R relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions R311K and Y313F relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions T214V and D314R relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions D314R and Y315M relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions R211K, R311K and D314K relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions R211K, T214V and Y313F relative to wild-type hA3B or hA3Bcrd, wherein the amino acid position is determined by reference to SEQ ID NO:19.

In some embodiments, the APOBEC3B deaminase mutant is derived from human APOBEC3B deaminase (hA3B) or C-terminal domain (hA3Bcrd) of human APOBEC3B deaminase, and comprises amino acid substitutions T214V, D314H and Y315M relative to wild-type hA3B or hA3Bcrd, wherein the amino acid position is determined by reference to SEQ ID NO:19.

In some specific embodiments, the APOBEC3B deaminase mutant comprises an amino acid sequence selected from SEQ ID NO:3-18, 26-31 and 32-34.

In some embodiments, the CRISPR effector protein is a "nuclease-inactivated CRISPR effector protein".

The "nuclease-inactivated CRISPR effector protein" refers to a CRISPR effector protein which loses double-stranded nucleic acid cleavage activity of the CRISPR effector protein but still maintains a DNA targeting ability guided by gRNA. The CRISPR effector protein without double-stranded nucleic acid cleavage activity also comprises a nickase which forms a nick on a double-stranded nucleic acid molecule, but does not completely cleave double-stranded nucleic acid.

In some preferred embodiments of the present invention, the nuclease-inactivated CRISPR effector protein of the present invention has nickase activity. Without being bound by any theory, it is believed that mismatch repair of eukaryotes directs the removal and repair of mismatched bases through nicks on DNA strands. U:G mismatch formed under the action of cytidine deaminase may be repaired into C:G. By introducing a nick on one strand containing unedited G, U:G mismatch can be preferably repaired into expected U:A or T:A.

In some embodiments, the nuclease-inactivated CRISPR effector protein is nuclease-inactivated Cas9. It has been known that the DNA cleavage domain of Cas9 nuclease contains two subdomains: an HNH nuclease subdomain and a RuvC subdomain. The HNH nuclease subdomain cleaves a strand complementary to gRNA, and the RuvC subdomain cleaves a strand that is not complementary to gRNA. Mutations in these subdomains can inactivate the nuclease of Cas9 to form "nuclease-inactivated Cas9". The nuclease-inactivated Cas9 still remains the DNA binding ability guided by gRNA. Therefore, in principle, when being fused with another protein, the nuclease-inactivated Cas9 can be simply co-expressed with proper guide RNA so as to target the another protein to almost any DNA sequences.

The nuclease-inactivated Cas9 of the present invention can be derived from different species of Cas9, for example, Cas9 (SpCas9) derived from S.pyogenes, or Cas9 (SaCas9) derived from S. aureus. Meanwhile, the HNH nuclease subdomain and RuvC subdomain of mutated Cas9 (for example, comprising mutated D10A and H840A) inactivate the nuclease of Cas9 to form nuclease dead Cas9 (dCas9). Mutation and inactivation of one of the subdomains can allow Cas9 to have nickase activity, so as to obtain a Cas9 nickase (nCase9), for example, nCas9 only having mutation D10A.

Therefore, in some embodiments of the present invention, the nuclease-inactivated Cas9 of the present invention contains amino acid substitutions D10A and/or H840A relative to wild-type Cas9.

In some specific embodiments of the present invention, the nuclease-inactivated Cas9 can also contain additional mutations. For example, nuclease-inactivated SpCas9 can also contain EQR, VQR or VRER mutation, and SpCas9 can also contain KKH mutation (Kim et al. Nat. Biotechnol. 35, 371-376.).

In some specific embodiments of the present invention, the nuclease-inactivated SpCas9 contains an amino acid sequence as shown in SEQ ID NO:35.

In some embodiments, the nuclease-inactivated CRISPR effector protein is nuclease-inactivated Cpfl. Cpfl contains one DNA cleavage domain (RuvC) which can be mutated to lose the DNA cleavage activity of Cpfl to form "Cpfl lacking DNA cleavage activity". The Cpfl lacking DNA cleavage activity still maintain the DNA binding ability guided by gRNA. Therefore, in principle, when being fused with another protein, the Cpfl lacking DNA cleavage activity can simply co-expressed with proper guide RNA so as to target the another protein to almost any DNA sequences.

The Cpfl lacking DNA cleavage activity of the present invention can be derived from different species of Cpfl, for example, Cpfl proteins derived from Francisella novicida U112, Acidaminococcus sp. BV3L6 and Lachnospiraceae bacterium ND2006, respectively called FnCpf1, AsCpfl and LbCpf1.

In some embodiments, the Cpfl lacking DNA cleavage activity is FnCpf1 lacking DNA cleavage activity. In some specific embodiments, the FnCpf1 lacking DNA cleavage activity contains D917A mutation relative to wild-type FnCpf1.

In some embodiments, the Cpfl lacking DNA cleavage activity is AsCpfl lacking DNA cleavage activity. In some specific embodiments, the AsCpfl lacking DNA cleavage activity contains D908A mutation relative to wild-type AsCpfl.

In some embodiments, the Cpfl lacking DNA cleavage activity is LbCpf1 lacking DNA cleavage activity. In some specific embodiments, the LbCpf1 lacking DNA cleavage activity contains D832A mutation relative to wild-type LbCpf1.

In some embodiments of the present invention, the APOBEC3B deaminase or APOBEC3B deaminase mutant is fused to the N terminal of the CRISPR effector protein (for example, nuclease-inactivated CRISPR effector protein, such as Cas9 or Cpfl).

In some embodiments of the present invention, the APOBEC3B deaminase or APOBEC3B deaminase mutant is fused to the CRISPR effector protein (for example, nuclease-inactivated CRISPR effector protein, such as Cas9 or Cpfl) through a linker. The linker can a non-functional amino acid sequence which has 1-50 (for example, 1, 2, 3, 4, 5, 6, 7, 8, 9, 10, 11, 12, 13, 14, 15, 16, 17, 18, 19, 20 or 20-25, 25-50) amino acids or more in length and no secondary or higher structure. For example, the linker can be a flexible linker. Preferably, the linker has 16 or 32 amino acids in length. In some specific embodiments, the linker is an XTEN linker as shown in SEQ ID NO:36 or 37.

In cells, the uracil DNA glycosylase catalyzes the removal of U from DNA and initiates base excision repair (BER) so as to cause U:G to be repaired into C:G. Therefore, without being bound by any theory, a uracil DNA glycosylase inhibitor contained in the base editing fusion protein of the present invention can increase the base editing efficiency.

Therefore, in some embodiments of the present invention, the base editing fusion protein also comprises a uracil DNA glycosylase inhibitor (UGI). In some specific embodiments, the uracil DNA glycosylase inhibitor comprises an amino acid sequence as shown in SEQ ID NO:38.

In some embodiments of the present invention, the base editing fusion protein of the present invention also contains a nuclear localization sequence (NLS). In general, one or more NLS in the base editing fusion protein should have enough intensity so as to drive the base editing fusion protein in the nucleus of a cell to realize the quantitative accumulation of the base editing function. In general, the intensity of nucleus localization activity is determined by the number and position of NLS in the base editing fusion protein, one or more specific NLS used, or a combination of these factors.

In some embodiments of the present invention, the NLS of the base editing fusion protein of the present invention can be located at N terminal and/or C terminal. In some embodiments of the present invention, the NLS of the base editing fusion protein of the present invention can be located between the APOBEC3B deaminase or APOBEC3B deaminase mutant and the CRISPR effector protein. In some embodiments, the base editing fusion protein comprises about 1, 2, 3, 4, 5, 6, 7, 8, 9, 10 or more NLS. In some embodiments, the base editing fusion protein comprises about 1, 2, 3, 4, 5, 6, 7, 8, 9, 10 or more NLS at or near N terminal. In some embodiments, the base editing fusion protein comprises about 1, 2, 3, 4, 5, 6, 7, 8, 9, 10 or more NLS at or near C terminal. In some embodiments, the base editing fusion protein comprises their combinations, for example, one or more NLS at N terminal and one or more NLS at C terminal. When more than one NLS is present, each NLS can be selected to be independent of other NLS. In some preferred embodiments of the present invention, the base editing fusion protein contains at least 2 NLS, for example, the at least 2 NLS are located at C terminal. In some embodiments, the NLS is located at the C terminal of the base editing fusion protein. In some embodiments, the base editing fusion protein contains at least 3 NLS.

In general, NLS is composed of one or more short sequences of positively charged lysine or arginine exposed to the surface of the protein, however, other types of NLS have been known as well. A non-limiting example of NLS includes PKKKRKV or KRPAATKKAGQAKKKK.

In some embodiments of the present invention, the N terminal of the base editing fusion protein contains NLS of an amino acid sequence as shown in PKKKRKV. In some embodiments of the present invention, the C terminal of the base editing fusion protein contains NLS of an amino acid sequence as shown in KRPAATKKAGQAKKKK. In some embodiments of the present invention, the C terminal of the base editing fusion protein contains NLS of an amino acid sequence as shown in PKKKRKV.

In addition, according to the DNA position required to be edited, the base editing fusion protein of the present invention can also contain other localization sequences, such as a cytoplasm localization sequence, a chloroplast localization sequence and a mitochondria localization sequence.

In another aspect, the present invention also provides use of the base editing fusion protein of the present invention in base editing of a target sequence in the genome of a cell.

In another aspect, the present invention also provides a system for base editing of a target sequence in the genome of a cell, comprising at least one of i)-v):
i) a base editing fusion protein according to the present invention, and guide RNA;
ii) an expression construct comprising a nucleotide sequence encoding the base editing protein according to the present invention, and a guide RNA;
iii) the base editing fusion protein according to the present invention, and an expression construct comprising a nucleotide sequence encoding a guide RNA;
iv) the expression construct comprising the nucleotide sequence encoding the base editing protein according to the present invention, and the expression construct comprising the nucleotide sequence encoding a guide RNA; and
v) an expression construct comprising the nucleotide sequence encoding the base editing fusion protein according to the present invention and the nucleotide sequence encoding a guide RNA;
wherein, the guide RNA is capable of targeting the base editing fusion protein to a target sequence in the genome of a cell.

As used herein, "base editing system" refers to a combination of components required for base editing of a genome in a cell or an organism. The individual components of the system, for example, the base editing fusion protein, or the one or more guide RNA, can be present independently, or can be present in a form of a composition in any combination.

As used herein, "guide RNA" and "gRNA" can be interchangeably used, which refers to a RNA molecule that can form a complex with the CRISPR effector protein and is capable of targeting the complex to a target sequence because it has a certain identity to the target sequence. The guide RNA targets the target sequence through base paring between the guide RNA and the complementary strand of the target sequence. For example, gRNA used by Cas9 nuclease or its functional mutant is often composed of crRNA and tracrRNA molecules that are partially complemented to form the complex, wherein crRNA contains a guide sequence (referred to as seed sequence) that has sufficient identity to the target sequence so as to be hybridized with the complementary strand of the target sequence and directs a CRISPR complex (Cas9+crRNA+tracerRNA) to specifically bind to the target sequence. However, it has been known in the art that single guide RNA (sgRNA) can be designed, which simultaneously contains the features of crRNA and tracrRNA. gRNA used by Cpfl nuclease or its functional mutant is often only composed of matured crRNA molecules, which is also referred to as sgRNA. Designing suitable gRNA based on the CRISPR effector protein as used and the target sequence to be edited is within the skill of those skilled person in the art.

In some embodiments, the base editing system of the present invention comprises more than one guide RNA, thereby more than one target sequence can be base edited simultaneously.

To obtain effective expression in the cell, in some embodiments of the present invention, the nucleotide sequence encoding the base editing base can be codon optimized against the organism from which the cells to be base edited are derived.

Codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the"Codon Usage Database" available at www.kazusa.orjp/codon/ and these tables can be adapted in a number of ways. See Nakamura, Y, et al."Codon usage tabulated from the international DNA sequence databases: status for the year 2000" Nucl. Acids Res. 28:292 (2000).

In some embodiments of the present invention, the guide RNA is a single guide RNA (sgRNA). A method for constructing suitable sgRNA according to a given target sequence has been known in the art. For example, see Wang, Y. et al. Simultaneous editing of three homoeoalleles in hexaploid bread wheat confers heritable resistance to powdery mildew. Nat. Biotechnol. 32, 947-951 (2014); Shan, Q. et al. Targeted genome modification of crop plants using a CRISPR-Cas system. Nat. Biotechnol. 31, 686-688 (2013); Liang, Z. et al. Targeted mutagenesis in Zea mays using TALENs and the CRISPR/Cas system. J Genet Genomics. 41, 63-68 (2014).

In some embodiments of the invention, the nucleotide sequence encoding the base-edited fusion protein and/or the nucleotide sequence encoding the guide RNA is operably linked to an expression control element, such as a promoter.

Examples of promoters that can be used in the present invention include, but are not limited to, polymerase (pol) I, pol II, or pol III promoters. Examples of pol I promoters include the chicken RNA pol I promoter. Examples of pol II promoters include, but are not limited to, the cytomegalovirus immediate early (CMV) promoter, the Rous sarcoma virus long terminal repeat (RSV-LTR) promoter, and the simian virus 40 (SV40) immediate early promoter. Examples of pol III promoters include U6 and H1 promoters. Inducible promoters such as the metallothionein promoter can be used. Other examples of promoters include T7 phage promoter, T3 phage promoter, β-galactosidase promoter, and Sp6 phage promoter. When used in plants, the promoter may be cauliflower mosaic virus 35S promoter, maize Ubi-1 promoter, wheat U6 promoter, rice U3 promoter, maize U3 promoter, rice actin promoter.

Organisms whose genomes can be modified by the base editing system of the present invention include any organism suitable for base editing, preferably eukaryotes. Examples of organisms include, but are not limited to, mammals such as humans, mice, rats, monkeys, dogs, pigs, sheep, cattle, cats; poultry such as chickens, ducks, geese; plants, including monocots and dicots, for example, the plants are crop plants including, but not limited to, wheat, rice, corn, soybean, sunflower, sorghum, canola, alfalfa, cotton, barley, millet, sugarcane, tomato, tobacco, cassava, and potato. Preferably, the organism is a plant. More preferably, the organism is rice.

### III. Method for producing genetically modified organisms

In another aspect, the present invention provides a method for producing a genetically modified organism, comprising introducing a base editing fusion protein of the invention or a expression construct comprising the base editing fusion protein of the invention, or a system of the present invention for base editing of a target sequence in the genome of a cell into a cell of the organism.

By introducing system of the present invention for base editing of a target sequence in the genome of a cell, the guide RNA targets the base-editing fusion protein to a target sequence in the genome of the cell of the organism, resulting in one or more C to T substitutions in the target sequence. In some preferred embodiments, the organism is a plant.

The design or selection of target sequences that can be recognized and targeted by the CRISPR effector protein and the guide RNA complex is within the skill of those skilled person in the art.

In some embodiments of the methods of the present invention, the method further comprises screening for an organism such as a plant containing the desired nucleotide substitution. Nucleotide substitutions in the organism such as a plant can be detected by T7EI, PCR/RE or sequencing methods, see e.g. Shan, Q., Wang, Y., Li, J. & Gao, C. Genome editing in rice and wheat using the CRISPR/Cas system. Nat. Protoc. 9, 2395-2410 (2014).

In the present invention, the target sequence to be modified may be located at any location in the genome, for example, in a functional gene such as a protein-encoding gene, or may be, for example, located in a gene expression regulatory region such as a promoter region or an enhancer region, thereby the gene functional modification or gene expression modification can be achieved.

In the methods of the present invention, the base editing system can be introduced into cells by a variety of methods well known to those skilled in the art. Methods that can be used to introduce a genome editing system of the present invention into a cell include, but are not limited to, calcium phosphate transfection, protoplast fusion, electroporation, lipofection, microinjection, viral infection (e.g., baculovirus, vaccinia virus, adenovirus, adeno-associated virus, lentivirus and other viruses), gene gun method, PEG-mediated protoplast transformation, Agrobacterium-mediated transformation.

A cell that can be edited by the method of the present invention can be a cell of mammals such as human, mouse, rat, monkey, dog, pig, sheep, cattle, cat; a cell of poultry such as chicken, duck, goose; a cell of plants including monocots and dicots, such as rice, corn, wheat, sorghum, barley, soybean, peanut and Arabidopsis thaliana and so on.

The methods of the invention are particularly suitable for producing genetically modified plants, such as crop plants. In the method of producing a genetically modified plant of the present invention, the base editing system can be introduced into a plant by various methods well known to those skilled in the art. Methods that can be used to introduce a base editing system of the invention into a plant include, but are not limited to, gene gun method, PEG-mediated protoplast transformation, Agrobacterium-mediated transformation, plant virus-mediated transformation, pollen tube pathway and ovary injection method.

In the method for producing a genetically modified plant of the present invention, the modification of the target sequence can be achieved by only introducing or producing the base-editing fusion protein and the guide RNA in the plant cell, and the modification can be stably inherited, without any need to stably transform the base editing system into plants. This avoids the potential off-target effect of the stable base editing system and also avoids the integration of the exogenous nucleotide sequence in the plant genome, thereby providing greater biosafety.

In some preferred embodiments, the introduction is carried out in the absence of selection pressure to avoid integration of the exogenous nucleotide sequence into the plant genome.

In some embodiments, the introduction comprises transforming the base editing system of the present invention into an isolated plant cell or tissue and then regenerating the transformed plant cell or tissue into an intact plant. Preferably, the regeneration is carried out in the absence of selection pressure, i.e., no selection agent for the selection gene on the expression vector is used during tissue culture. Avoiding the use of a selection agent can increase the regeneration efficiency of the plant, obtaining a modified plant free of exogenous nucleotide sequences.

In other embodiments, the base editing system of the present invention can be transformed into specific parts of an intact plant, such as leaves, shoot tips, pollen tubes, young ears or hypocotyls. This is particularly suitable for the transformation of plants that are difficult to regenerate in tissue culture.

In some embodiments of the invention, the in vitro expressed protein and/or the in vitro transcribed RNA molecule are directly transformed into the plant. The protein and/or RNA molecule is capable of performing base editing in plant cells and is subsequently degraded by the cell, avoiding integration of the exogenous nucleotide sequence in the plant genome.

Thus, in some embodiments, genetic modification and breeding of plants using the methods of the present invention may result in plants free of integration of exogenous DNA, i.e., transgene-free modified plants. In addition, the base editing system of the present invention has high specificity (low off-target rate) for base editing in plants, which also improves biosafety.

Plants that can be base-edited by the methods of the invention include monocots and dicots. For example, the plant may be a crop plant such as wheat, rice, corn, soybean, sunflower, sorghum, canola, alfalfa, cotton, barley, millet, sugar cane, tomato, tobacco, tapioca or potato.

In some embodiments of the present invention, the target sequence is associated with a plant trait, such as an agronomic trait, whereby the base editing results in a plant having altered traits relative to a wild type plant.

In the present invention, the target sequence to be modified may be located at any position in the genome, for example, in a functional gene such as a protein-encoding gene, or may be, for example, located in a gene expression regulatory region such as a promoter region or an enhancer region, thereby gene functional modification or gene expression modification can be achieved. Accordingly, in some embodiments of the present invention, the substitution of C to T results in an amino acid substitution in the target protein. In other embodiments of the present invention, the substitution of C to T results in a change in expression of the target gene.

In some embodiments of the present invention, the method further comprises obtaining progeny of the genetically modified plant.

In another aspect, the present invention provides a genetically modified plant or a progeny thereof, or a part thereof, wherein the plant is obtained by the method of the invention described above. In some embodiments, the genetically modified plant or a progeny thereof, or a part thereof is transgene-free.

In another aspect, the present invention provides a method of plant breeding comprising crossing a genetically modified first plant obtained by the above method of the present invention with a second plant not containing the genetic modification, thereby the genetic modification is introduced into the second plant.

### Examples

For the sake of understanding the present invention, the present invention will be described in detail by reference to relevant specific embodiments and accompanying drawings below. The accompanying drawings give preferred embodiments of the present invention. However, the present invention can be implemented in many different forms, but is not limited to embodiments described herein. In contrast, the purpose of providing these embodiments is to more easily and more thoroughly understanding the contents disclosed in the present invention.

### Example 1 Selection of A3Bctd mutation sites based on protein structure information

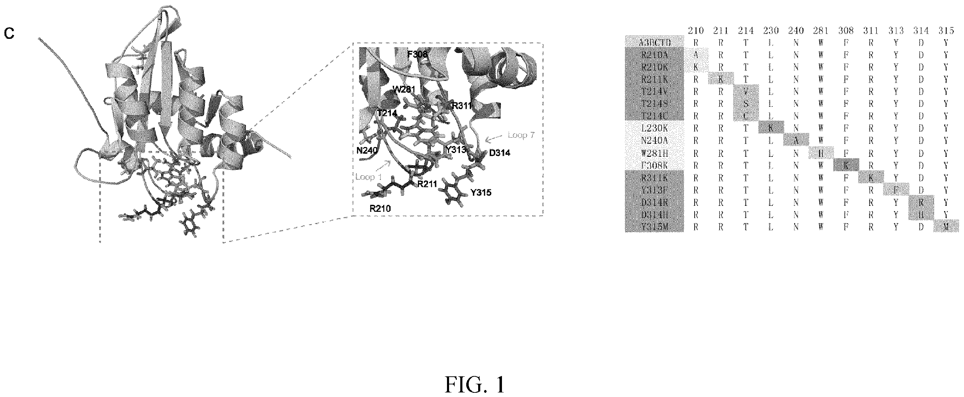

According to the published structure information (PDB:2NBQ) of hA3Bctd and the published structure information (PDB: 5CQD, 5CQH and 5TD5) of full-length hAPOBEC3B, amino acid point mutations were performed on key loop regions Loop1 and Loop7 closely associated with the binding of hA3Bctd to single-stranded DNA to reduce the ability of binding to single-stranded DNA. Point mutation positions and types of specific amino acids are as shown in Fig. 1.

Candidate base editing systems were optimized on an A3A-BE3 vector skeleton (SEQ ID NO:1, comprising a base editor of human APOBEC3A), the APOBEC3A sequence in the A3A-BE3 vector was replaced with an artificially synthesized A3Bctd DNA fragment (SEQ ID NO:2) with Gbison method to obtain an A3Bctd-BE3 vector. In the A3A-BE3 vector, point mutations were carried out on encoding amino acids of A3Bctd by utilizing fused PCR and Gbison method to respectively obtain point mutation base editing vectors of A3Bctd-R210A-BE3, A3Bctd-R210K-BE3, A3Bctd-R211K-BE3, A3Bctd-T214C-BE3, A3Bctd-T214G-BE3, A3Bctd-T214S-BE3, A3Bctd-T214V-BE3, A3Bctd-L230K-BE3, A3Bctd-N240A-BE3, A3Bctd-W281H-BE3, A3Bctd-F308K-BE3, A3Bctd-R311K-BE3, A3Bctd-Y313F-BE3, A3Bctd-D314R-BE3, A3Bctd-D314H-BE3 and A3Bctd-Y315M-BE3 (deaminase amino acid sequences after point mutation are respectively as shown in SEQ ID NO: 3-18).

In addition, constructed control plasmids are A3A-BE3, YEE-BE3, RK-BE3, eA3A-BE3, A3A-R128A-BE3, A3A-Y130F-BE3 and untruncated APOBEC3B-BE3 (wherein, deaminase sequences are seen in SEQ ID NO:19-25), wherein YEE and RK are two mutants of APOBEC1 deaminase on a BE3 vector, which were constructed by fused PCR and Gbison method. The sequences of A3A deaminase iswas artificially synthesized, and R128A and Y130F of A3A were constructed by fused PCR and Gbison method.

### Example 2 Verification of editing efficiency and specificity of A3Bctd-BE3 system carrying single point mutation on protoplasts

### 2.1 Construction of vector

Guide RNA vectors used in this experiment include pSp-sgRNA and pSa-sgRNA vectors. 8 targets in Table 1 were respectively constructed, wherein the target of -T1 was constructed to the pSp-sgRNA vector using a digestion and ligation method to serve as a guide RNA vector for detecting the on target efficiency, the target at the end of -SaT1 or -SaT2 was constructed to the pSa-sgRNA vector using the digestion and ligation method to serve as a vector for detecting the off target ability using a TA-AS method.

The principle of the TA-AS method is to co-transfect a to-be-detected base editing system (such as a base editing system based on nSpCas9 in this experiment) with other CRISPR systems such as a nSpCas9 system that are orthogonal (i.e., those that cannot share gRNA) to the to-be-detected base editing system and can create single-stranded regions so that the orthogonal other CRISPR systems create one long-term stable single-stranded region at a selected site in the genome. If the to-be-detected base editing system has a genome-wide random off target effect, deamination will be performed on C base in this single-stranded region and unexpected editing will be caused. The random off target effect of the base editing system can be efficiently and simply detected by high-throughput sequencing of amplicons at selected sites.

**Table 1**

| sgRNA | Target sequence | Oligo-F | Oligo-R |
|---|---|---|---|
| *OsAAT1*-T1 | CAAGGATCCCAGCCCC GTGAAGG | GGCGCAAGGATCCC AGCCCCGTGA | AAACTCACGGGGC TGGGATCCTTG |
| *OsACTG*-T1 | ATCATCCGCCACGACG GCGGCGG | GGCGATCATCCGCCA CGACGGCGG | AAACCCGCCGTCG TGGCGGATGAT |
| *OsEV*-T1 | ACACACACACTAGTAC CTCTGGG | GGCGACACACACAC TAGTACCTCT | AAACAGAGGTACT AGTGTGTGTGT |
| *OsCDC48*-T1 | GACCAGCCAGCGTCT GGCGCCGG | GGCGGACCAGCCAG CGTCTGGCGC | AAACGCGCCAGAC GCTGGCTGGTC |
| *OsCDC48-*SaT1 | CTCGTTCCCATGTCATT GTCATGGGT | GGCGCTCGTTCCCAT GTCATTGTC | AAACGACAATGAC ATGGGAACGAG |
| *OsDEP1*-SaT1 | GGTCACTCAGCCTGCA GTACTGAAT | GGCGGGTCACTCAG CCTGCAGTA | AAACTACTGCAGG CTGAGTGACC |
| *OsDEP1*-SaT2 | GTCGTGCCCTGAATGT TCCTGTGGGT | GGCGGTCGTGCCCTG AATGTTCCT | AAACAGGAACATT CAGGGCACGAC |
| *OsNRT1.1B*-SaT1 | CGATCATCGACAGGTC GGCGGCGGAGT | GGCGCGATCATCGAC AGGTCGGCGG | AAACCCGCCGACC TGTCGATGATCG |

### 2.2 Protoplast transformation

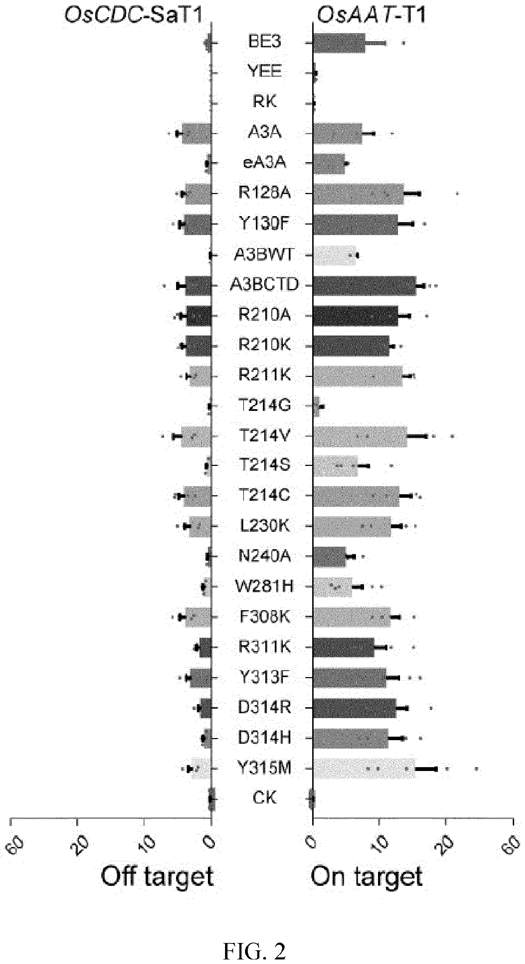

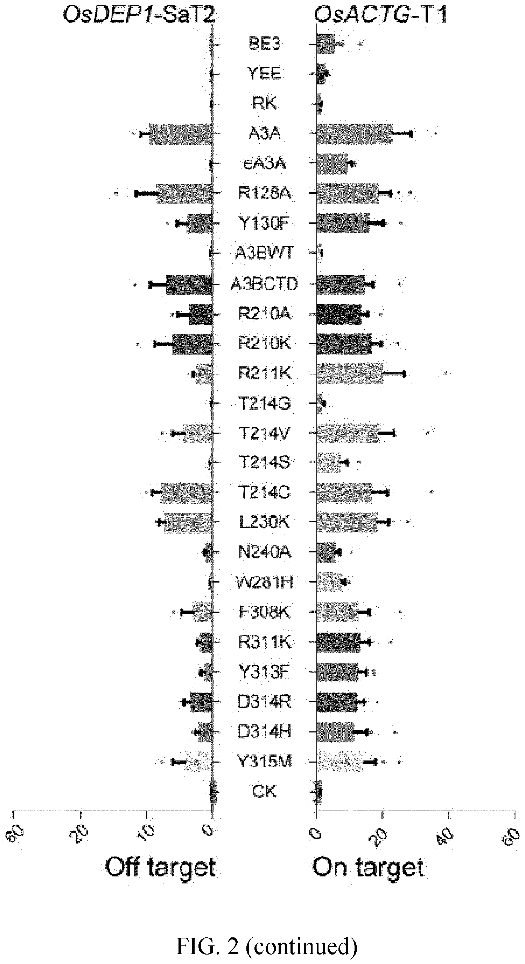

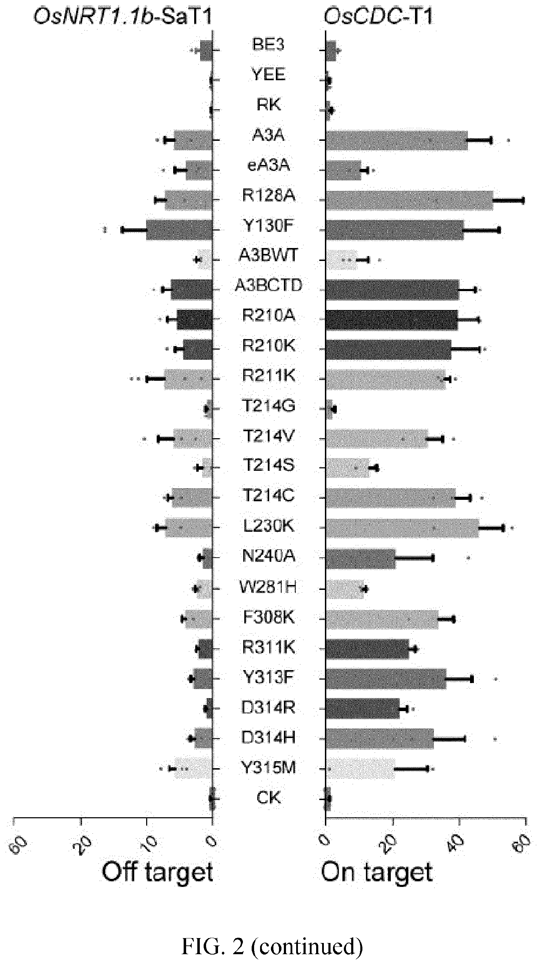

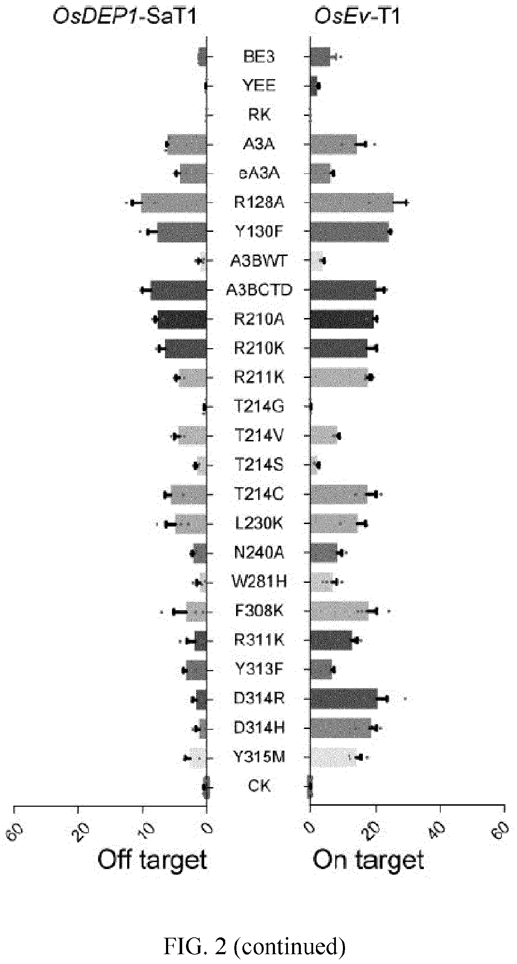

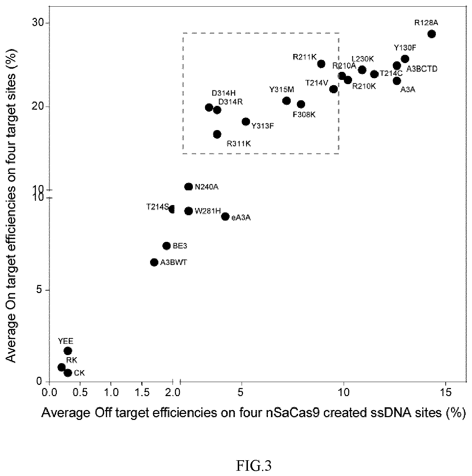

By using conventional BE3, A3A-BE3, YEE-BE3, RK-BE3, eA3A-BE3, A3A-R128A-BE3, A3A-Y130F, untruncated APOBEC3B-BE3 and A3Bctd-BE3 systems as control, each base editing system together with its own guide RNA vector pSp-sgRNA and pnSaCsa9 in a TA-AS system as well as corresponding pSa-sgRNA were co-transformed into rice protoplast, target site amplicon sequencing was carried out after culture for 2 days, average values of four target sites and 4 off target sites were taken to evaluate the on target efficiency and the off target efficiency. Each target of each base editing system had at least three bilological repetitions, and results are as shown in Fig.2 and Fig.3. It was found that eight point mutations R211K, T214V, F308K, R311K, Y313F, D314R, D314H and Y315M can reduce the off target efficiency while maintaining relatively high mutation efficiency, wherein seven point mutations are located on Loop1 and Loop7. These seven mutants were combined to further improve the specificity.

### Example 3 Further improvement of specificity by combination of the point mutations obtained by screening

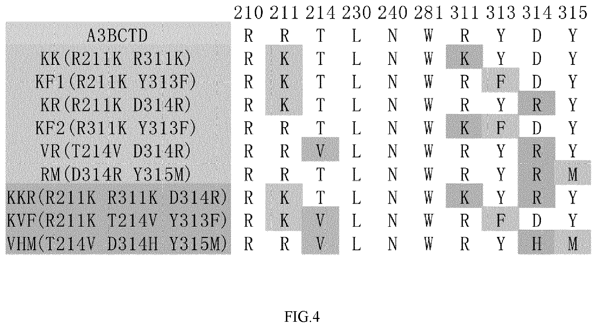

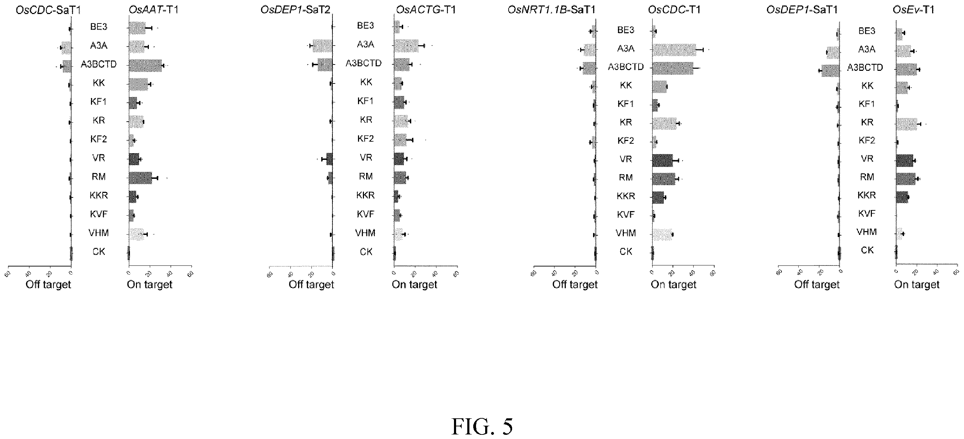

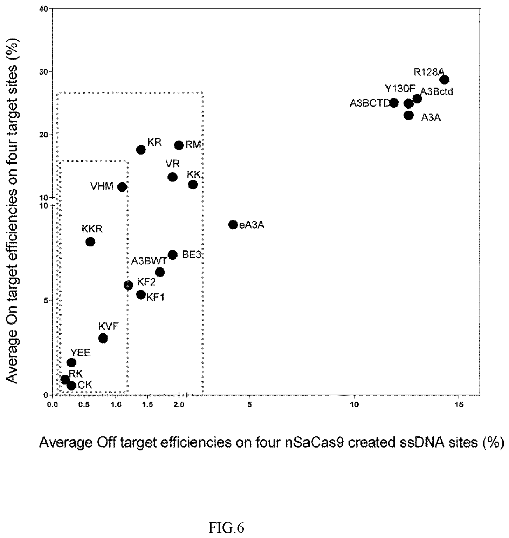

The seven amino acid mutation sites screened in the former step were combined to form nine double mutants and triple mutants (Fig.4). Similar to the above experimental flow chart, four target sites and four off target sites were tested for target site mutation efficiency and off target efficiency of the combined variants (Fig.4). It was found by test results that two triple mutants KKR and VHM had reduced the off target efficiency to a level equivalent to the background while maintaining high on target efficiency (Fig.5 and Fig.6). Especially for KKR mutant, TS-AS system detection results showed that the average off target efficiency of the detected four targets was only 0.6%, which was reduced by 21 times compared with that of wild-type A3Bctd (Fig.5 and Fig.6).

### Example 4 Analysis on editing characteristics of new cytosine base editing systems

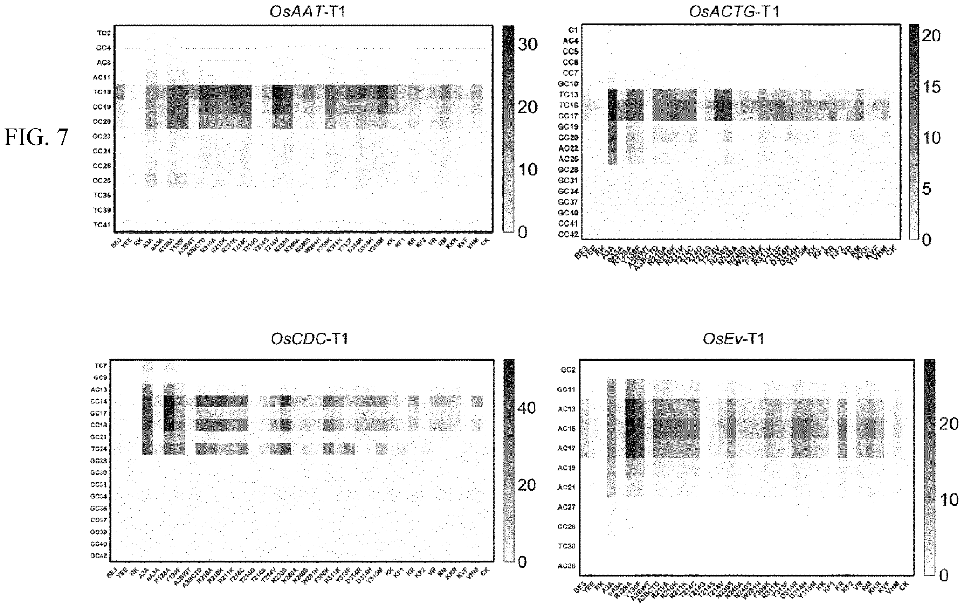

Mutant characteristics, including editing window, preference and editing product types, of all the base editing systems in four target sites were analyzed (PAM sequence is considered as positions 21-23). In the aspect of editing window, it can be found that the editing efficiency of A3Bctd was equivalent to the editing efficiencies of A3A-BE3, A3A-R128A and A3A-Y130F, but its working window was narrower than the working windows of A3A-BE3, A3A-R128A and A3A-Y130F. The single amino acid mutants A3Bctd-Y313F, A3Bctd-211K, A3Bctd-Y315M and A3Bctd-T214V can reduce the size of the working window to 2-3 bp. However, the double mutant or triple mutant can reduce the size of the working window to 1-2 bp while slightly scarifying the editing efficiency (Fig.7).

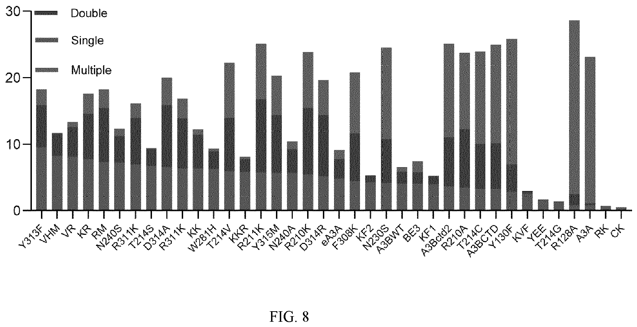

The gene editing product can be divided into single, double and multiple mutation types according to the number of mutated Cs. Fig.8 depicts average mutation types of editing products of all base editing systems in this experiment in four target sites. By ranking according to the mutation efficiency of single C, it can be found that although the total efficiency of the A3A-BE3 series editing systems is relatively high, the ratio of the produced single C mutation products was extremely low, and the probability of obtaining single C mutation products was extremely small. The probability of the mutant Y313F of A3Bctd to obtain an editing product with only one C mutation was approximately 10%, and VHM, VR and KR similarly showed relatively high editing accuracy. It is noted that VHM and KKR mutants had extremely high product accuracy, which can basically generate editing products with only one C mutation or two C mutations.

## Claims

1. A base editing fusion protein, comprising an APOBEC3B deaminase or a APOBEC3B deaminase mutant fused with a CRISPR effector protein.

2. The base editing fusion protein according to claim 1, wherein the APOBEC3B deaminase mutant is or is derived from a human APOBEC3B deaminase, for example, the human APOBEC3B deaminase comprises an amino acid sequence as shown in SEQ ID NO:19.

3. The base editing fusion protein according to claim 1, wherein the APOBEC3B deaminase mutant is or is derived from a C-terminal domain (hA3Bcrd) of a human APOBEC3B deaminase, for example, the hA3Bcrd comprises an amino acid sequence as shown in SEQ ID NO:2.

4. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at one or more of position 210, position 211, position 214, position 230, position 240, position 281, position 308, position 311, position 313, position 314 and position 315 relative to wild-type hA3B or hA3Bcrd, wherein the amino acid position is determined by reference to SEQ ID NO:19.

5. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at one or more of position 211, position 214, position 308, position 311, position 313, position 314 and position 315 relative to wild-type hA3B or hA3Bcrd, wherein the amino acid position is determined by reference to SEQ ID NO:19.

6. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 211 and position 311 relative to wild-type hA3B or hA3Bcrd, wherein the amino acid position is determined by reference to SEQ ID NO:19.

7. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at one or more of position 211 and position 313 relative to wild-type hA3B or hA3Bcrd, wherein the amino acid position is determined by reference to SEQ ID NO:19.

8. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 211 and position 314 relative to wild-type hA3B or hA3Bcrd, wherein the amino acid position is determined by reference to SEQ ID NO:19.

9. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 311 and position 313 relative to wild-type hA3B or hA3Bcrd, wherein the amino acid position is determined by reference to SEQ ID NO:19.

10. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 214 and position 314 relative to wild-type hA3B or hA3Bcrd, wherein the amino acid position is determined by reference to SEQ ID NO:19.

11. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 314 and position 315 relative to wild-type hA3B or hA3Bcrd, wherein the amino acid position is determined by reference to SEQ ID NO:19.

12. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 211, position 311 and position 314 relative to wild-type hA3B or hA3Bcrd, wherein the amino acid position is determined by reference to SEQ ID NO:19.

13. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 211, position 214 and position 313 relative to wild-type hA3B or hA3Bcrd, wherein the amino acid position is determined by reference to SEQ ID NO:19.

14. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions at position 214, position 314 and position 315 relative to wild-type hA3B or hA3Bcrd, wherein the amino acid position is determined by reference to SEQ ID NO:19.

15. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises one or more amino acid substitutions selected from R210A, R210K3, R211K, T214C, T214G, T214S, T214V, L230K, N240A, W281H, F308K, R311K, Y313F, D314R, D314H and Y315M relative to wild-type hA3B or hA3Bcrd, wherein the amino acid position is determined by reference to SEQ ID NO:19.

16. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises one or more amino acid substitutions selected from R211K, T214V, F308K, R311K, Y313F, D314R, D314H and Y315M relative to wild-type hA3B or hA3Bcrd, wherein the amino acid position is determined by reference to SEQ ID NO:19.

17. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions R211K and R311K relative to wild-type hA3B or hA3Bcrd, wherein the amino acid position is determined by reference to SEQ ID NO:19.

18. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises one or more amino acid substitutions R211K and Y313F relative to wild-type hA3B or hA3Bcrd, wherein the amino acid position is determined by reference to SEQ ID NO:19.

19. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions R211K and D314R relative to wild-type hA3B or hA3Bcrd, wherein the amino acid position is determined by reference to SEQ ID NO:19.

20. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions R311K and Y313F relative to wild-type hA3B or hA3Bcrd, wherein the amino acid position is determined by reference to SEQ ID NO:19.

21. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions T214V and D314R relative to wild-type hA3B or hA3Bcrd, wherein the amino acid position is determined by reference to SEQ ID NO:19.

22. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions D314R and Y315M relative to wild-type hA3B or hA3Bcrd, wherein the amino acid position is determined by reference to SEQ ID NO:19.

23. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions R211K, R311K and D314K relative to wild-type hA3B or hA3Bcrd, wherein the amino acid position is determined by reference to SEQ ID NO:19.

24. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions R211K, T214V and Y313F relative to wild-type hA3B or hA3Bcrd, wherein the amino acid position is determined by reference to SEQ ID NO:19.

25. The base editing fusion protein according to claim 2 or 3, wherein the APOBEC3B deaminase mutant is derived from human APOBEC3B deaminase (hA3B) or C-terminal domain (hA3Bcrd) of human APOBEC3B deaminase, and comprises amino acid substitutions T214V, D314H and Y315M relative to wild-type hA3B or hA3Bcrd, wherein the amino acid position is determined by reference to SEQ ID NO:19.

26. The base editing fusion protein according to claim 1, wherein the APOBEC3B deaminase mutant comprises an amino acid sequence selected from SEQ ID NO:3-18, 26-31 and 32-34.

27. The base editing fusion protein according to any one of claims 1-26, wherein the CRISPR effector protein is a nuclease-inactivated CRISPR effector protein such as a CRISPR nickase.

28. The base editing fusion protein according to claim 27, wherein the nuclease-inactivated CRISPR effector protein is a nuclease-inactivated Cas9 which comprises amino acid substitutions D10A and/or H840A relative to wild-type Cas9, for example, the nuclease-inactivated Cas9 comprises an amino acid sequence as shown in SEQ ID NO:35.

29. The base editing fusion protein according to any one of claims 1-28, wherein the APOBEC3B deaminase or APOBEC3B deaminase mutant is fused to the N terminal of the CRISPR effector protein.

30. The base editing fusion protein according to any one of claims 1-29, wherein the APOBEC3B deaminase or APOBEC3B deaminase mutant is fused to the CRISPR effector protein through a linker, for example, the linker is a linker as shown in SEQ ID NO:36 or 37.

31. The base editing fusion protein according to any one of claims 1-30, wherein the base editing fusion protein also comprising a uracil DNA glycosylase inhibitor (UGI), for example, the uracil DNA glycosylase inhibitor comprises an amino acid sequence as shown in SEQ ID NO:38.

32. The base editing fusion protein according to any one of claims 1-31, the base editing fusion protein also comprises a nuclear localization sequence (NLS).

33. A system for base editing of a target sequence in a cell genome, comprising at least one of i)-v):
i) a base editing fusion protein according to any one of claims 1-32, and a guide RNA;
ii) an expression construct containing a nucleotide sequence encoding the base editing protein according to any one of claims 1-32, and a guide RNA;
iii) the base editing fusion protein according to any one of claims 1-32, and an expression construct containing a nucleotide sequence encoding a guide RNA;
iv) the expression construct containing the nucleotide sequence encoding the base editing protein according to any one of claims 1-32, and the expression construct containing the nucleotide sequence encoding a guide RNA; and
v) an expression construct containing the nucleotide sequence encoding the base editing fusion protein according to any one of claims 1-32 and the nucleotide sequence encoding a guide RNA;
wherein, the guide RNA is capable of targeting the base editing fusion protein to a target sequence in the genome of a cell.

34. The system according to claim 33, comprising more than one guide RNA or expression constructs thereof, whereby more than one target sequence can be base-edited simultaneously.

35. The system according to claim 33 or 34, wherein the nucleotide sequence encoding the base editing fusion protein is codon optimized against the organism from which the cells to be base edited are derived.

36. The system according to any one of claims 33-35, wherein the guide RNA is a single guide RNA (sgRNA).

37. The system according to any one of claims 33-36, wherein the nucleotide sequence encoding the base editing fusion protein and/or the nucleotide sequence encoding the guide RNA is operatively linked to an expression regulation element such as promoter.

38. A method for producing a genetically modified organism, comprising: introducing a base editing fusion protein according to any one of claims 1-32, or an expression construct containing a nucleotide sequence encoding the base editing fusion protein according to any one of claims 1-32, or a system for base editing of a target sequence in the genome of a cell according to any one of claims 33-36 into a cell of the organism.

39. The method according to claim 38, wherein the organism is a plant.
